# EUROPEAN PATENT APPLICATION

(11) **EP 3 415 145 A2**
(43) Date of publication of application: **19.12.2018**
(21) Application number: 18177239.3
(22) Date of filing: 12.06.2018
(51) Int. Cl.: A61K 31/36, A61K 31/575, A61P 25/00

(54) **METHOD FOR TREATING STROKE OR REDUCING NERVE INJURY**

(30) Priority: 12.06.2017 US 201715620055
(71) Applicant: Arjil Biotech Holding Company Limited, 30013 Hsinchu (TW)
(72) Inventor: WU, Yeh B, Taipei City 10491 (TW); LO, Jir-Mehng, Hsinchu County 30641 (TW); SHIH, Ying Chu, Nantou County 55760 (TW); LIANG, Hui Ju, Taipei City 10076 (TW)
(74) Representative: Hoefer & Partner Patentanwälte mbB

(57) **Abstract**

The disclosure is related to a compound for use in treating stroke or reducing nerve injury, wherein the compound is isolated and purified from *Antrodia camphorata* fruiting body by methanol extraction. In particular, the compound is dehydroeburicoic acid, dehydrosulphurenic acid (dehydrosulfurenic acid) or 4,7-Dimethoxy-5-methyl-1,3-benzodioxole.

## Description

### FIELD OF THE INVENTION

The present invention pertains to a method for treating stroke or reducing nerve injury.

### BACKGROUND OF THE INVENTION

Cerebrovascular accident (CVA), commonly known as stroke, refers to the rapid loss of brain function caused by abnormality in supplying the brain with blood. The most common factors are thrombus, embolism, and hemorrhage. It is caused by the destruction of the brain's blood supply, making the brain cells unable to get enough nutrients and oxygen, resulting in nerve function injury. The stroke can be divided into hemorrhage stroke and ischemia stroke. Both ischemic stroke and hemorrhagic stroke may cause brain dysfunction. Hemorrhagic stroke comes from intracerebral hemorrhage, usually having higher mortality. Ischemic stroke comes from cerebral ischemia caused by brain thrombosis and brain embolism, which has lower mortality in usual but easily leading to injury on neurobehavioral ability. Common symptoms of stroke include incapable of moving unilateral limbs or unilateral body anesthesia, unable to understand words from other people, unable to speak, feeling dizzy, losing unilateral vision and so on. Stroke patients may also have long-term sequelae such as pneumonia and urinary incontinence.

The main risk factor of stroke is high blood pressure. Other factors include age, history of stroke, transient ischemic heart disease, diabetes, high cholesterol, smoking, atrial fibrillation etc. Therefore, common drugs nowadays for treating and preventing strokes are anticoagulants (e.g. Warfarin TAB, COFaRin TAB, Dabigatran), antiplatelet agent (e.g. Aspirin, Clopidogrel, Ticlopidine, Dipyridamole, Aggrenox), brain metabolism improved agents (Pentoxifylline, gingko extract, Piracetam, Nicametate), anticoagulants, hypotensive agents, statins and so on.

In view of the side effects of the above-mentioned drugs, those skilled in the art are actively looking for alternative drugs which are low in biological toxicity, having fewer side effects, and having ability to protect nerve injury of the brain after stroke. For example, a Chinese Patent, CN 101406569 B, discloses a pharmaceutical composition for treating cerebrovascular disease using a traditional Chinese medicine. US Patent No. 9333207 B2 discloses the use of "1-adamantylethyloxy-3-morpholino-2-propanol" for treating cerebrovascular disease and neurodegenerative diseases in the central nervous system. Taiwan Patent No. 1461204 discloses the efficacy of *Antrodia camphorata* in treating stroke. US Patent No. 8486460 B2 discloses a Chinese herbal medicine composition for reducing the likelihood of stroke and a method for treating a stroke.

It is still desired to develop a new method/pharmaceutical composition for treating stroke with no side effect and low toxicity.

### BRIEF SUMMARY OF THE INVENTION

The present invention provides a new method for treating stroke or reducing nerve injury. The method comprises administering to a subject in need thereof a pharmaceutical composition comprising a therapeutically effective amount of a compound selected from the group consisting of the following: 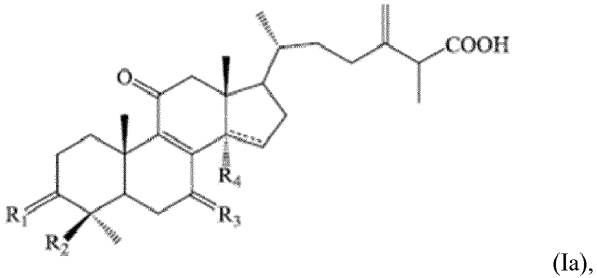 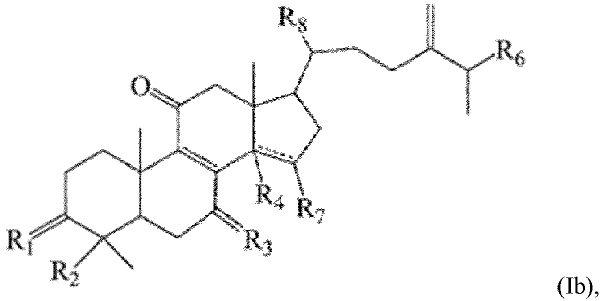 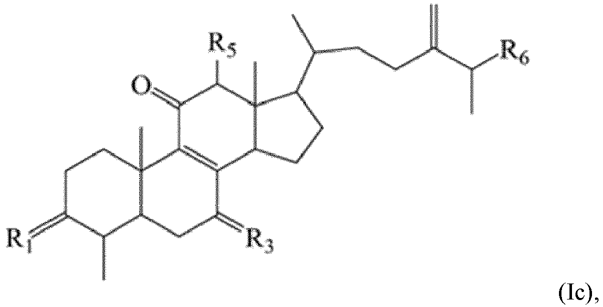 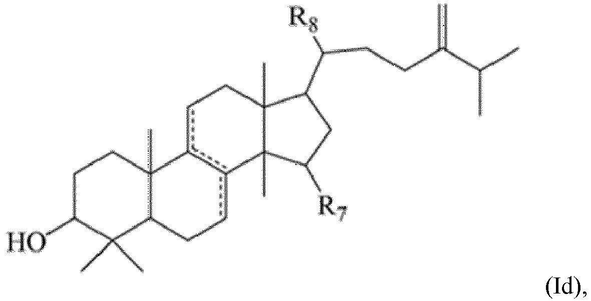 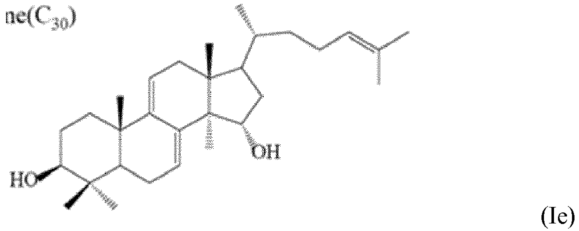 or 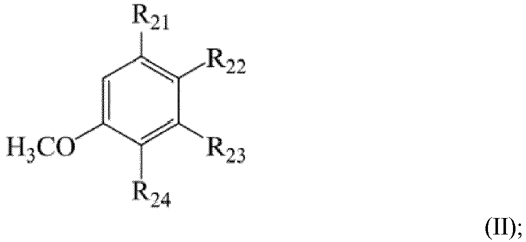 wherein R₁ is O, α-OH or β-H; R₂ is H or OH; R₃ is O, α-H, β-OAc or H₂; R₄ is H or OH; R₅ is H, or OH; R₆ is COOH or COO(CH₂)n-CH₃; n is an integer from 0-3; R₇ is H, OH or OAc; R₈ is CH₃ or COOH; R₂₁ is CH₃, COOH, or COO(CH₂)n-CH₃; n is an integer from 0-3; each of R₂₂, R₂₃, R₂₄ is OCH₃, or R₂₂ and R₂₃ together form a O-CH₂-O; or R₂₃ and R₂₄ together form a O-CH₂-O; the dotted line represents a single bond or a double bond.

In one particular example of the present invention, the compound of formula (Ia) is dehydroeburicoic acid:

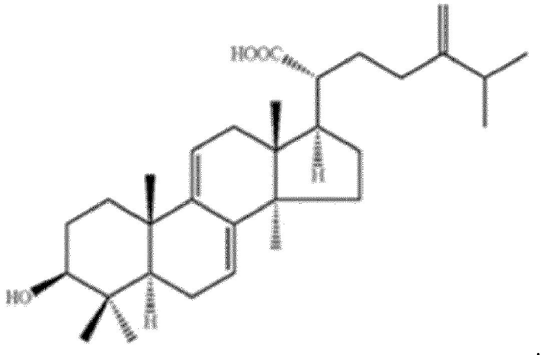

In another example of the present invention, the compound of formula (Ia) is dehydrosulphurenic acid (dehydrosulfurenic acid):

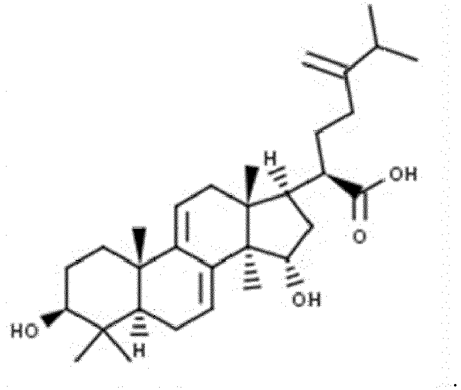

In one particular example of the present invention, the compound of formula (II) is 4,7-Dimethoxy-5-methyl-1,3-benzodioxole

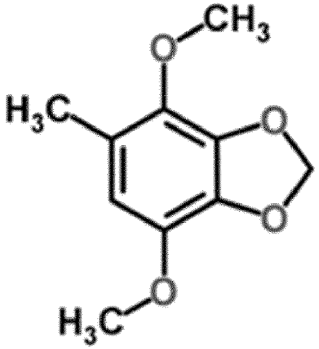

In one embodiment of the present invention, the compound of formula (Ib) is antcin A:

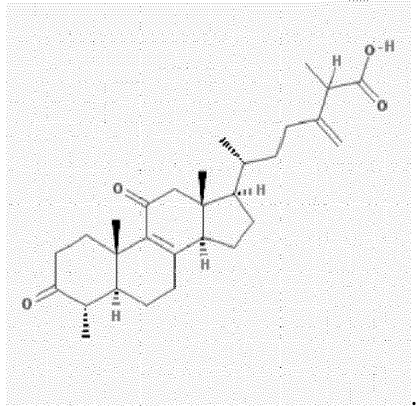

In one embodiment of the present invention, the compound of formula (Ib) isantcin B:

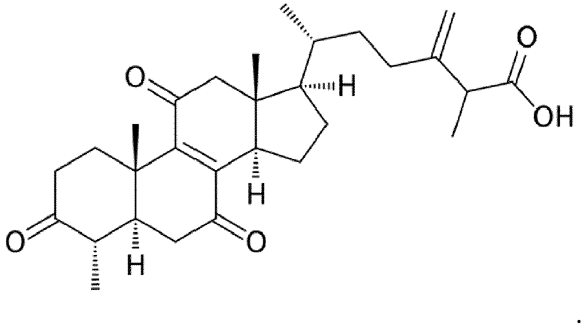

In one embodiment of the present invention, the compound of formula (Ib) isantcin C:

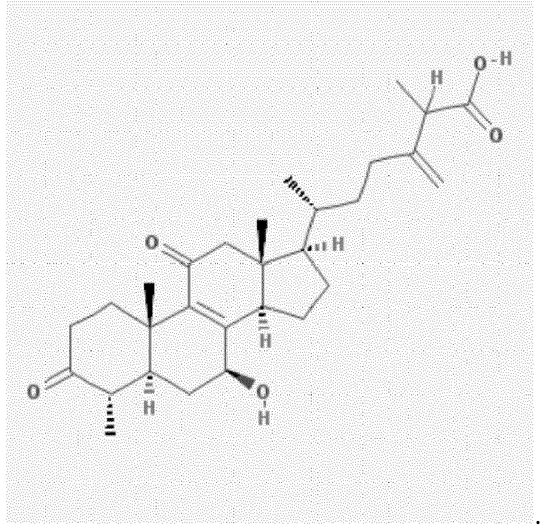

In one embodiment of the present invention, the compound of formula (Ib) is antcin H:

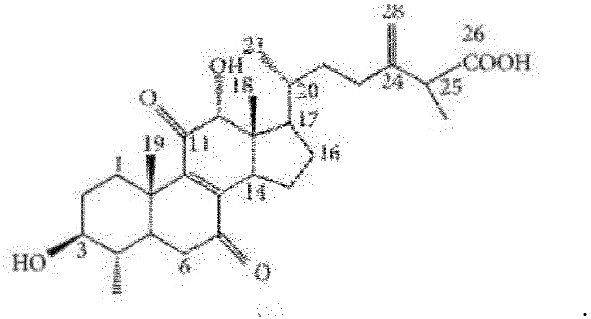

In one embodiment of the present invention, the compound of formula (Ib) is antcin K:

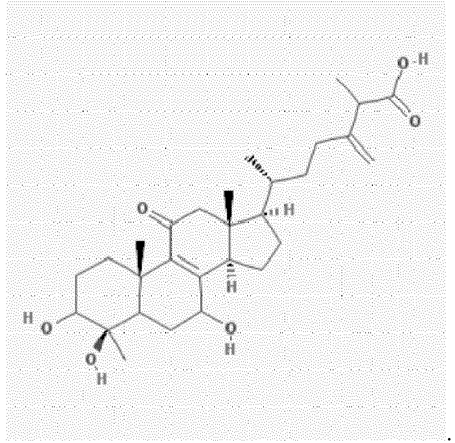

In one preferred embodiment of the present invention, the stroke is ischemia stroke.

In further aspect, the invention also provides a use of a compound for manufacturing a medicament for treating stroke or reducing nerve injury, wherein the compound has the formula of (Ia), (Ib), (Ic), (Id), (Ie) or (II) as defined above.

In yet aspect, the invention provides a pharmaceutical composition for use in treating stroke or reducing nerve injury, which comprises a therapeutically effective amount of the compound of formula (Ia), (Ib), (Ic), (Id), (Ie) or (II) as defined above and one or more pharmaceutically acceptable carriers.

It is to be understood that both the foregoing general description and the following detailed description are exemplary and explanatory only and are not restrictive of the invention.

### BRIEF DESCRIPTION OF THE SEVERAL VIEWS OF THE DRAWINGS

The foregoing summary, as well as the following detailed description of the invention, will be better understood when read in conjunction with the appended drawings. For the purpose of illustrating the invention, there are shown in the drawings embodiments which are presently preferred.

In the drawings:

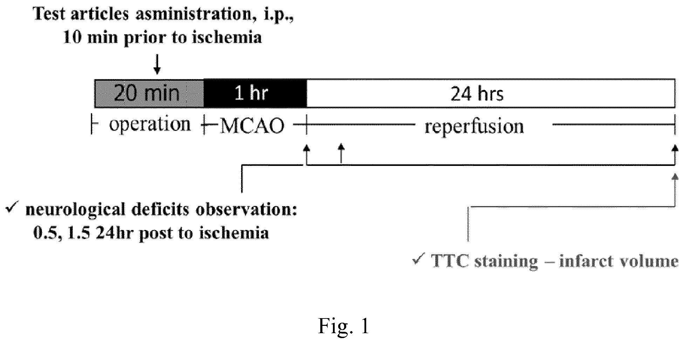

Fig. 1 is a time flow chart of administration of a therapeutic compound 10 minutes before inducing ischemic stroke (MCAO surgery) in one embodiment of the present invention, wherein neurobehavioral analyses are performed at 0.5, 1.5 and 24 hours postoperatively.

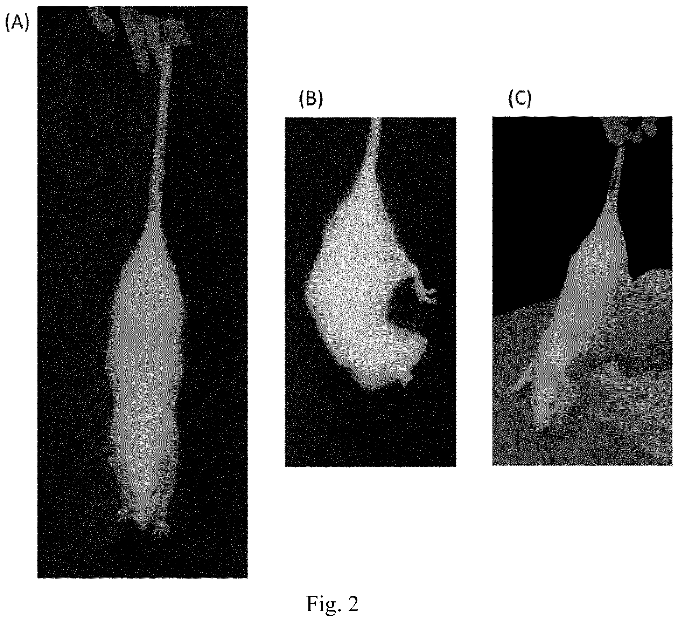

Fig. 2 is a schematic diagram of neurobehavioral analysis methods for determining the degree of nerve injury of rats in one embodiment of the present invention.

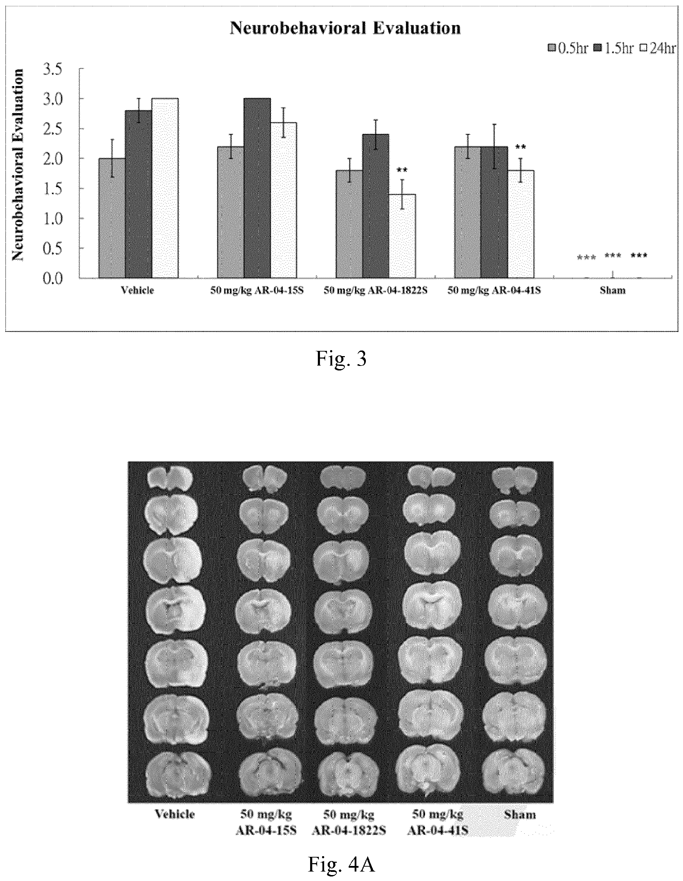

Fig. 3 shows the results of neurobehavioral analyses performed at 0.5, 1.5, and 24 hours after MCAO surgery on each control group and experimental group in one embodiment of the present invention, wherein data are expressed as mean ± standard deviation with ** indicating p value < 0.01, *** indicating p value < 0.001, and the number of samples being 3-5.
Fig. 4A is a cross-sectional view of a brain in one embodiment of the present invention. The brain is sliced into seven pieces from the front end to the range of 15 mm, each having thickness of 2 mm. The forefront (1 mm) of the brain is removed.

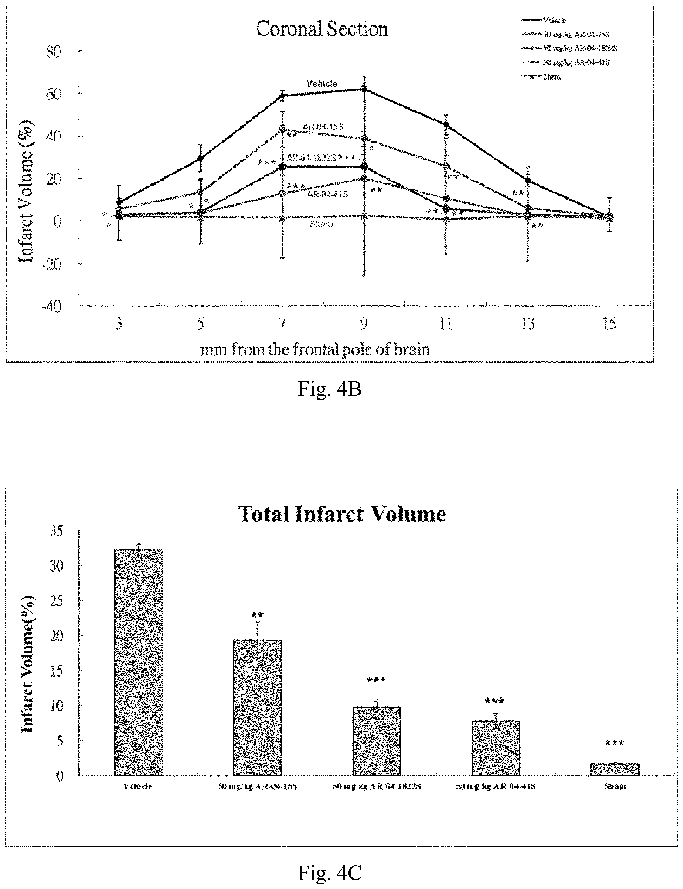

Fig. 4B shows the brain infarct site and the percentage of brain infarct volume of each compound and control group according to the embodiment in Fig. 4A, and the area of the infarct brain is analyzed from the front end to the range of 15 mm, wherein data are expressed as mean ± standard deviation with * indicating p value <0.05, ** indicating p value <0.01, *** indicating p value <0.001, and the number of samples being 3-5.
Fig. 4C shows the total infarct volume (%) of the brain for each compound and control group according to the embodiment in Fig. 4A, and the area of the infarct brain is analyzed from the front end to the range of 15 mm, wherein data are expressed as mean ± standard deviation with ** indicating p value <0.01, *** indicating p value <0.001, and the number of samples being 3-5.

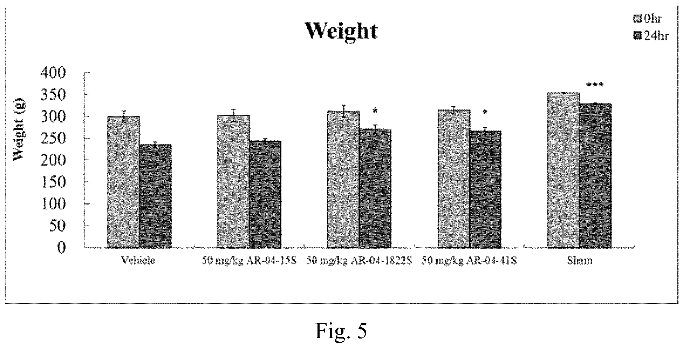

Fig. 5 shows an analytical chart of body weight changes in each control and experimental group before MCAO surgery and 24 hours after surgery in one embodiment of the present invention, wherein data are expressed as mean ± standard deviation with * indicating p value <0.05, *** Indicating p value <0.001, and the number of samples being 3-5.

### DETAILED DESCRIPTION OF THE INVENTION

Unless defined otherwise, all technical and scientific terms used herein have the same meaning as commonly understood by a person skilled in the art to which this invention belongs.

The present invention provides the use of a compound for preparing a medicament for treating stroke or reducing nerve injury. The compound is selected from the group consisting of the following:
(1) 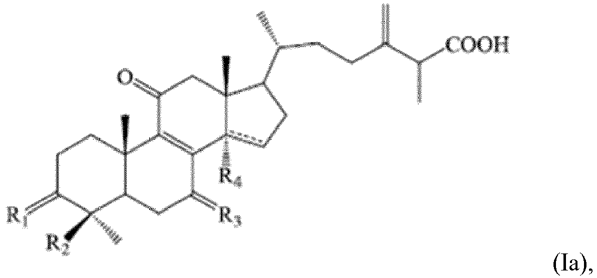 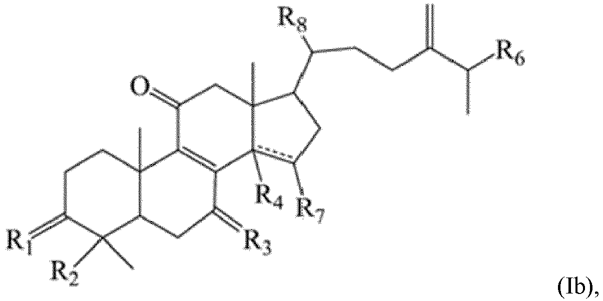 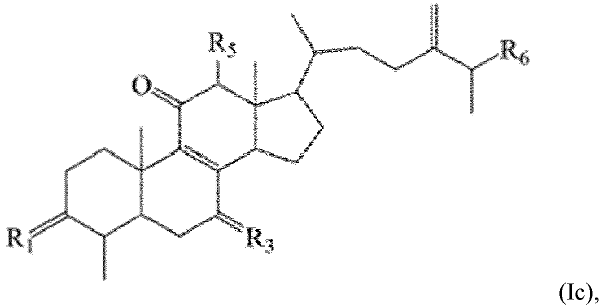 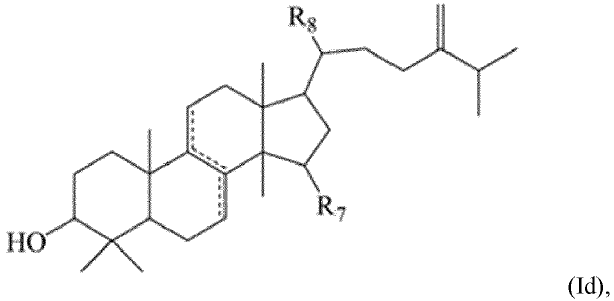 or 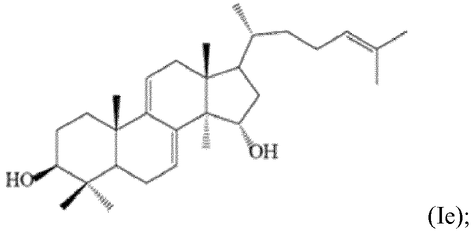 wherein R₁ is O, α-OH or β-H; R₂ is H or OH; R₃ is O, α-H, β-OAc or H₂; R₄ is H or OH; R₅ is H or OH; R₆ is COOH or COO(CH₂)n-CH₃; R₇ is H, OH, or OAc; R₈ is CH₃ or COOH; the dotted line represents a single bond or a double bond; n is an integer from 0-3; or
(2) 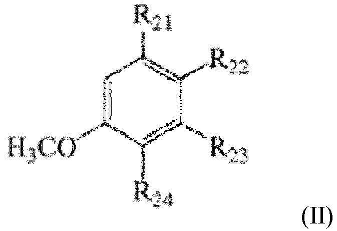 wherein R₂₁ is CH₃ or COOH, or COO(CH₂)n-CH₃; n is an integer from 0-3; each of R₂₂, R₂₃, R₂₄ is OCH₃, or R₂₂ and R₂₃ together form a O-CH₂-O; or R₂₃ and R₂₄ together form a O-CH₂-O.

In the embodiment of the invention, the compound may be:

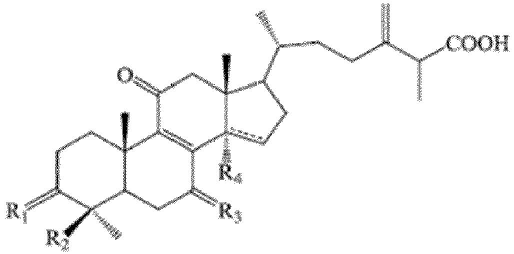

| | R₁ | R₂ | R₃ | R₄ | Δ |
|---|---|---|---|---|---|
| Antcin A | O | H | H₂ | H | |
| Antcin B | O | H | O | H | |
| Antcin C | O | H | β-OH | H | |
| Antcin D | O | H | O | OH | |
| Antcin E | O | H | H₂ | | 14 |
| Antcin F | O | H | β-OP | | 14 |
| Antcin K | α-OH | OH | β-OH | H | |

In another embodiment of the invention, the compound may be

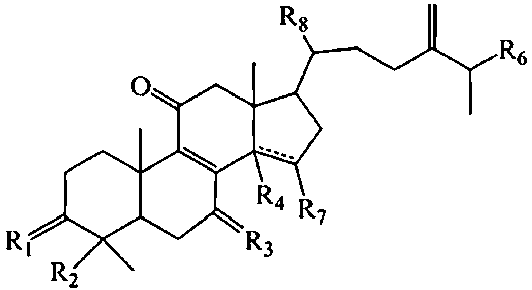

| | R₁ | R₄ | R₅ | R₆ |
|---|---|---|---|---|
| Zhankuic acid B | β-H | H | H | COOH |
| | α-OH | | | |
| Zhankuic acid C | β-H | H | OH | COOH |
| | α-OH | | | |
| Zhankuic acid D | O | H | H | COOEt |
| Zhankuic acid E | β-H | H | OH | COOEt |
| | α-OH | | | |

In one yet embodiment of the invention, the compound may be

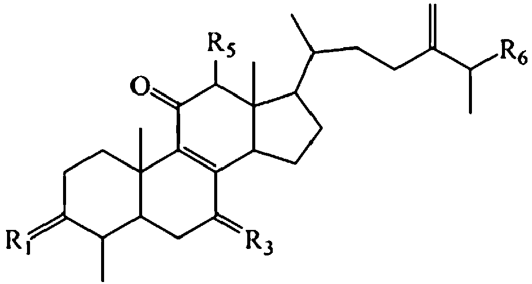

| | R₁ | R₃ | R₅ |
|---|---|---|---|
| methyl antcinate B | O | O | H |
| methyl autcinate G | O | β-OAc | H |
| | | α-H | |
| methyl antcinate H | β-H | O | OH |
| | α-OH | | |
| | O | H₂ | H |

R=COOMe

In further embodiment of the invention, the compound may be

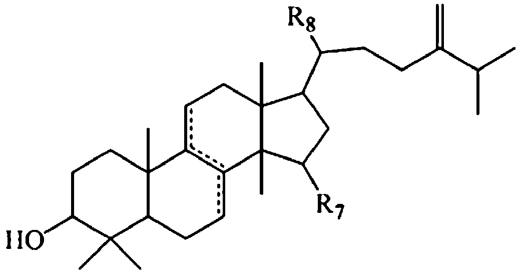

| | R₇ | R₈ | Δ |
|---|---|---|---|
| dehydroeburicoic acid | H | COOH | 7.9(11) |
| eburicol | H | CH₃ | 8 |
| 15a-acetyl-dehydrosulphurenic acid | OAc | COOH | 7.9(11) |
| dehydrosulphurenic acid | OH | COOH | 7.9(11) |
| eburicoic acid | H | COOH | 8 |
| Versisponic acid | OAc | COOH | 8 |
| sulphurenic acid | OH | COOH | 8 |

In one particular embodiment of the invention, the compound may be lanostane:

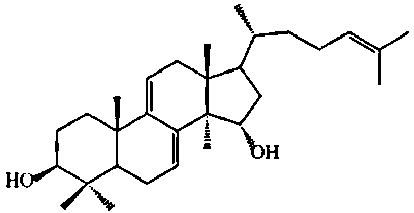

In addition, the compound of formula (II) may be

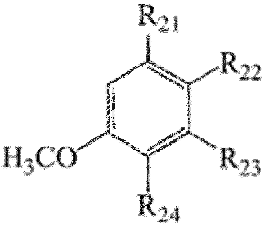

| | R₂₁ | R₂₂ | R₂₃ | R24 |
|---|---|---|---|---|
| | CH₃ | OCH₃ | -O-CH₂O- | |
| | COOCH₃ | OCH₃ | -O-CH₂-O- | |
| | COOH | -O-CH₂-O- | | OCH₃ |

According to the invention, the compound of formula (Ia) is selected from the group consisting of: 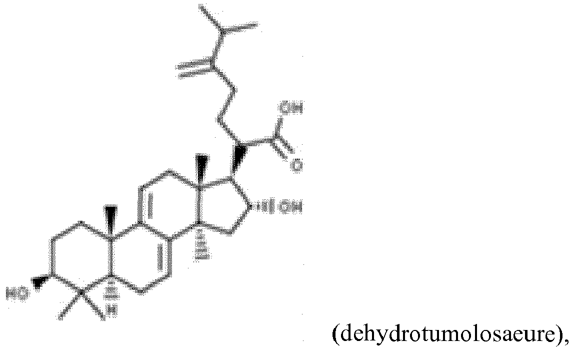 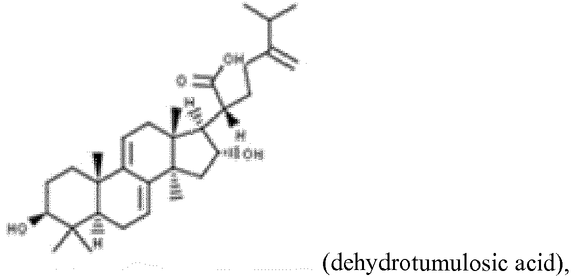 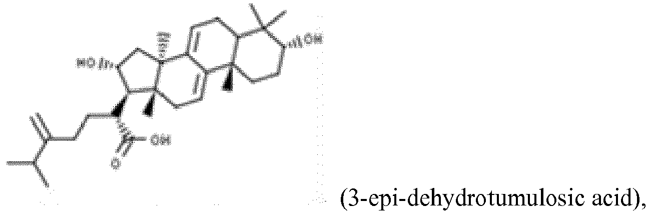 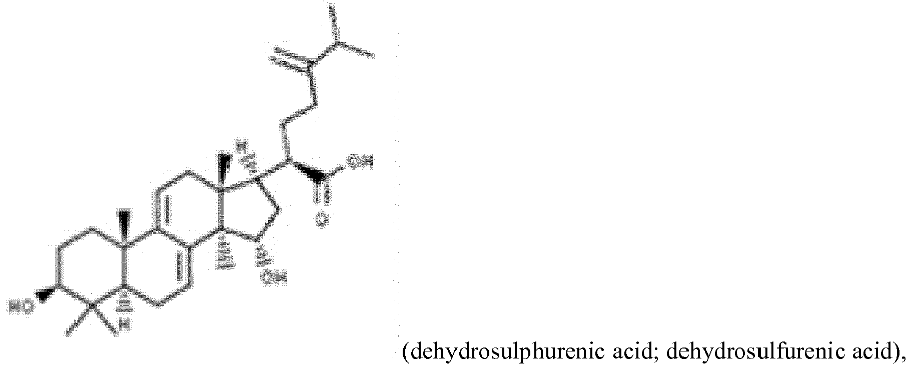 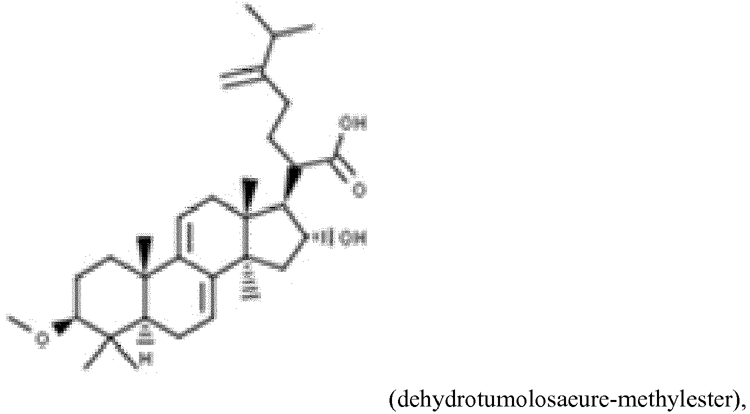 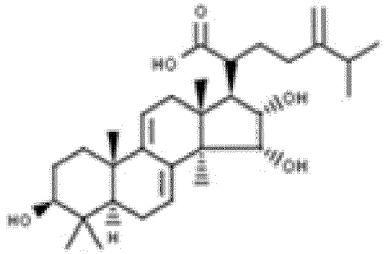 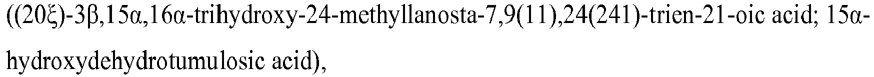 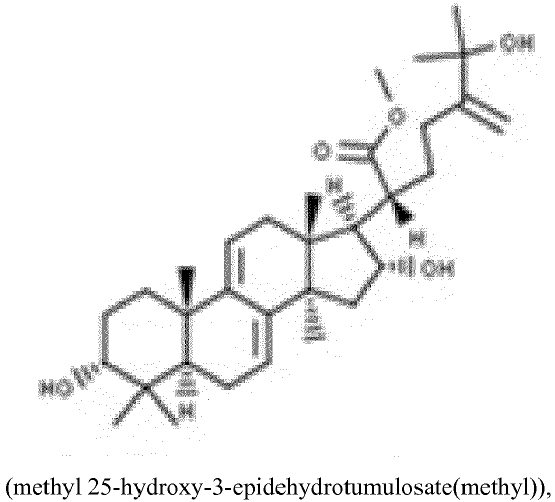 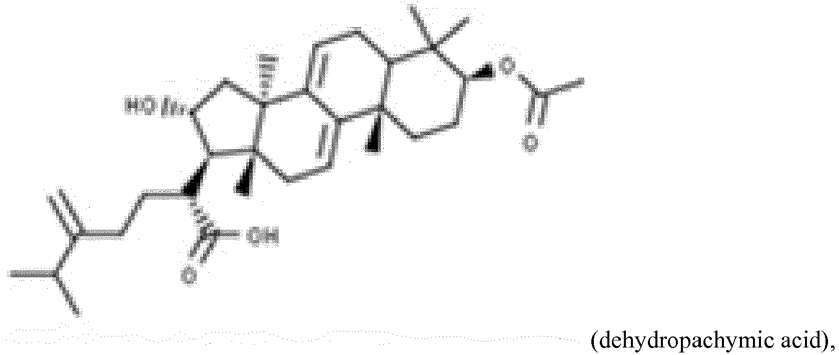 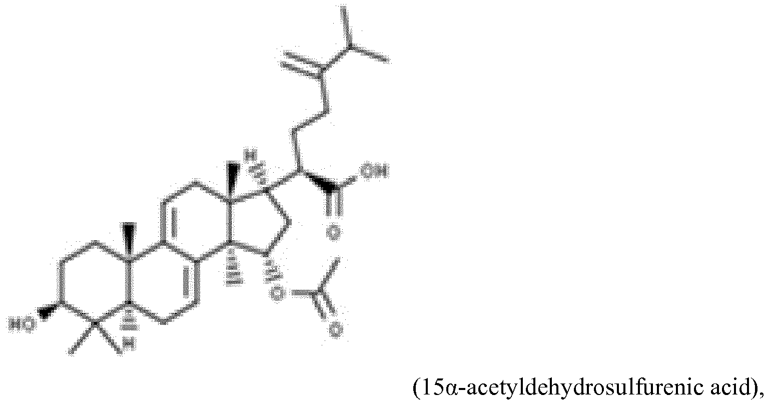 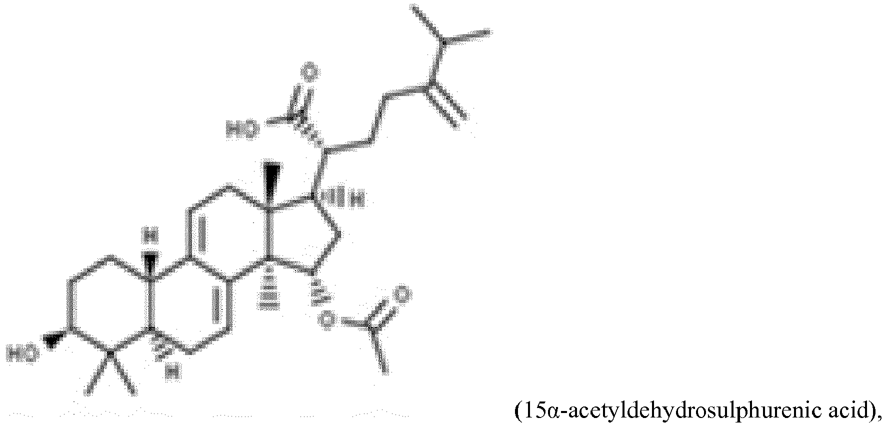 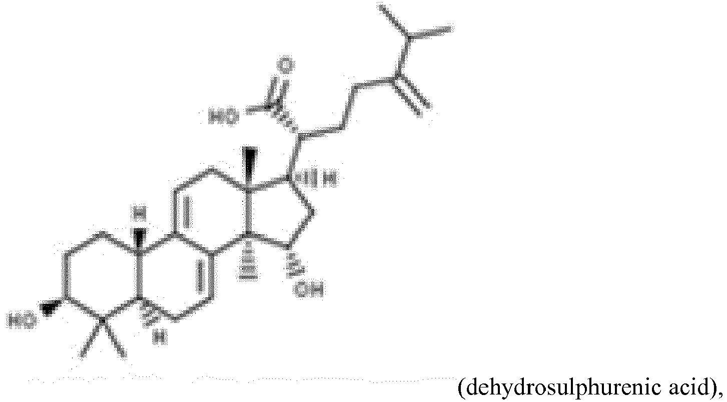 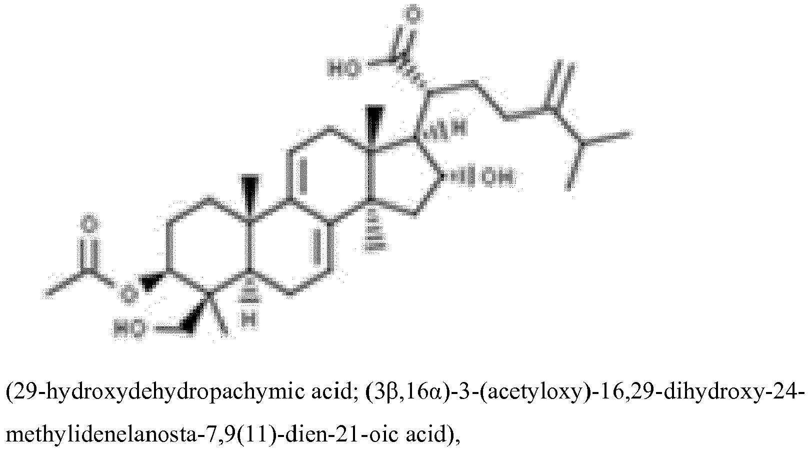 and

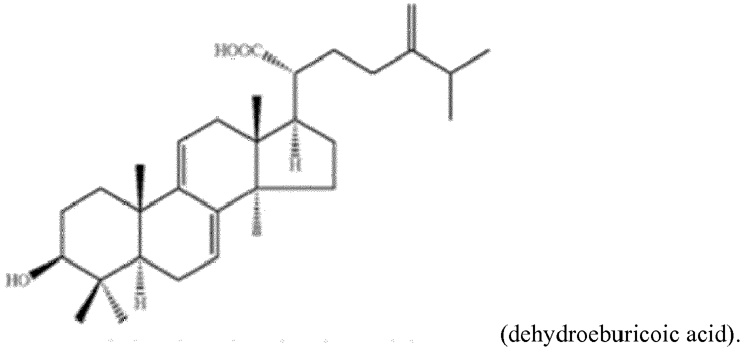

According to the invention, the compound of formula (Ib) is selected from the group consisting of 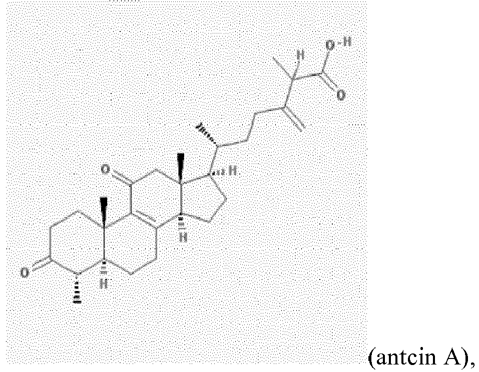 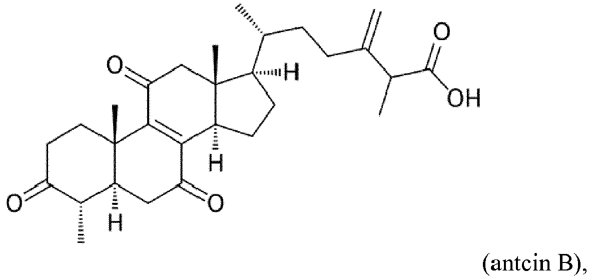 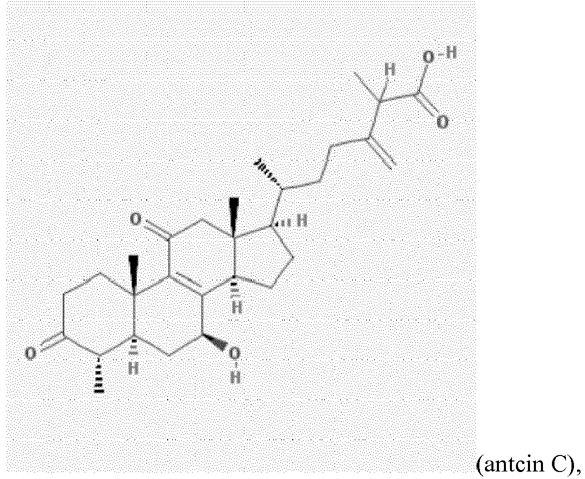 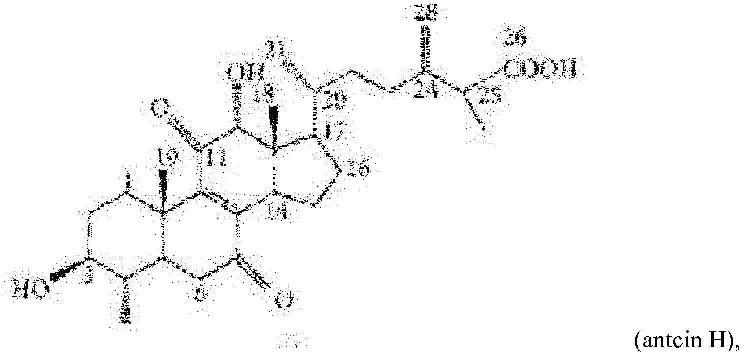 and

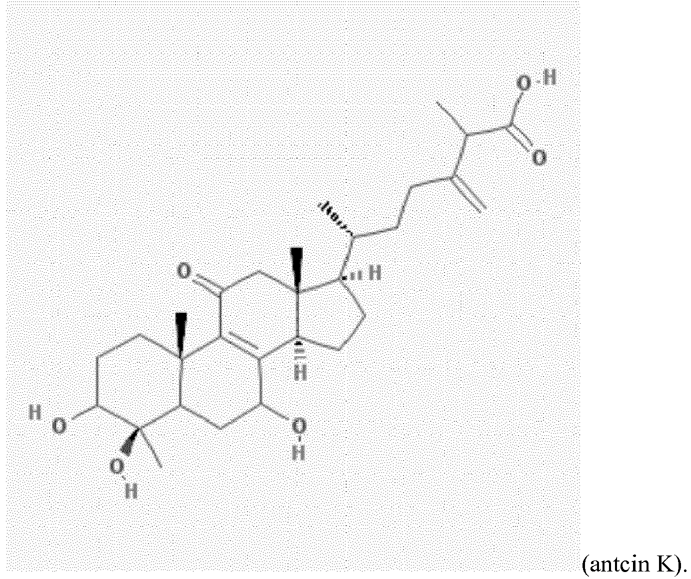

In one particular example of the invention, the compound of formula (II) is

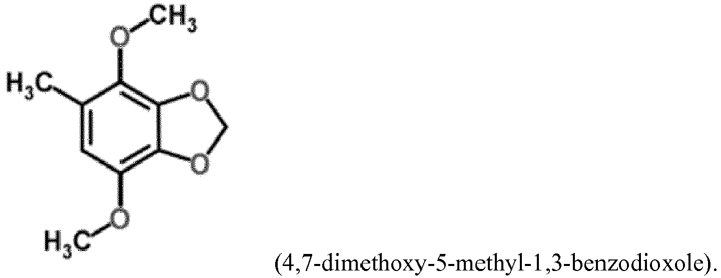

In the invention, the compound is proved to be effective for treating stroke, particularly ischemic stroke, and reducing nerve injury.

In the particular examples of the invnetion, dehydroeburicoic acid, 4,7-Dimethoxy-5-methyl-1,3-benzodioxole, or dehydrosulphurenic acid (dehydrosulfurenic acid) provided significant effects in treating stroke and reducing nerve injury.

Accordingly, the invention provides the use of the compound of formula (Ia), (Ib), (Ic), (Id), (Ie) or (II) for manufacturing a medicament for treating stroke or reducing nerve injury.

On the other hand, the invention provides a method for treating stroke or reducing nerve injury. The method comprises administering to a subject in need thereof a pharmaceutical composition comprising a therapeutically effective amount of the compound of formula (Ia), (Ib), (Ic), (Id), (Ie) or (II).

The term "therapeutically effective amount" as used herein refers to an amount of a compound or pharmaceutical agent which, as compared to a corresponding subject who has not received such amount, results in an effect in treatment, healing, prevention, or amelioration of a disease, disorder, or side effect, or a decrease in the rate of advancement of a disease or disorder. The term also includes within its scope amounts effective to enhance normal physiological function.

For use in therapy, the therapeutically effective amounts of the compound is formulated as a pharmaceutical composition for administration. Accordingly, the invention further provides a pharmaceutical composition comprising a therapeutically effective amount of the compound of formula (Ia), (Ib), (Ic), (Id), (Ie) or (II) and one or more pharmaceutically acceptable carriers.

The term "pharmaceutically acceptable carriers" used herein refers to a carrier(s), diluent(s) or excipient(s) that is acceptable, in the sense of being compatible with the other ingredients of the formulation and not deleterious to the subject to be administered with the pharmaceutical composition. Any carrier, diluent or excipient commonly known or used in the field may be used in the invention, depending to the requirements of the pharmaceutical formulation.

According to the invention, the pharmaceutical composition may be adapted for administration by any appropriate route, including but not limited to oral, rectal, nasal, topical, vaginal, or parenteral route. In one particular example of the invention, the pharmaceutical composition is formulated for oral administration. Such formulations may be prepared by any method known in the art of pharmacy.

The present invention is further illustrated by the following examples, which should be construed as illustrative only and not in any way limit the remainder of the present invention. Without further illustration, it is believed that those skilled in the art will be able to make the best use of the present invention based on the description herein.

### Preparation Examples

### Example 1. Preparation of Active ingredients: dehydroeburicoic acid and 4,7-Dimethoxy-5-methyl-1,3-benzodioxole

100 grams of *Antrodia camphorata* fruiting body was heat-recirculated with methanol for 6 hours, and the extract was collected and dried under reduced pressure to obtain 15 grams of the antrodia camphorate methanol extract.

Fifteen (15) grams of the *Atrodia camphorate* methanol extract as obtained above was taken, filled with silicon dioxide, and subjected to a gradient elution with the eluant "hexane / ethyl acetate / methanol" in a column separation (3x12 cm) to obtain dehydroeburicoic acid (No.: AR-04-41S), 4,7-Dimethoxy-5-methyl-1,3-benzodioxole (No.: AR-04-15S), and dehydrosulphurenic acid (dehydrosulfurenic acid) (No.: AR-04-1822S).

It was found that the three compounds below were obtained:
dehydroeburicoic acid (No. AR-04-41S) having the following formula: 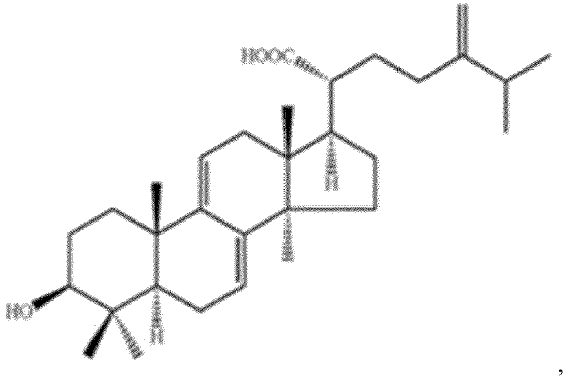
4,7-Dimethoxy-5-methyl-1,3-benzodioxole (No. AR-04-15S) having the following formula: 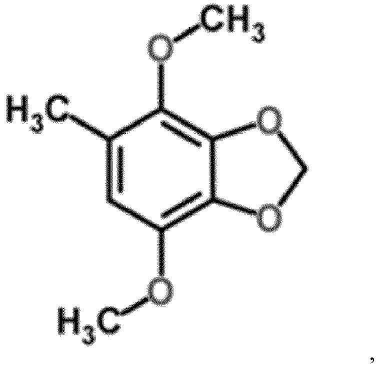 and
dehydrosulphurenic acid (dehydrosulfurenic acid) (No. AR-04-1822S) having the following formula:

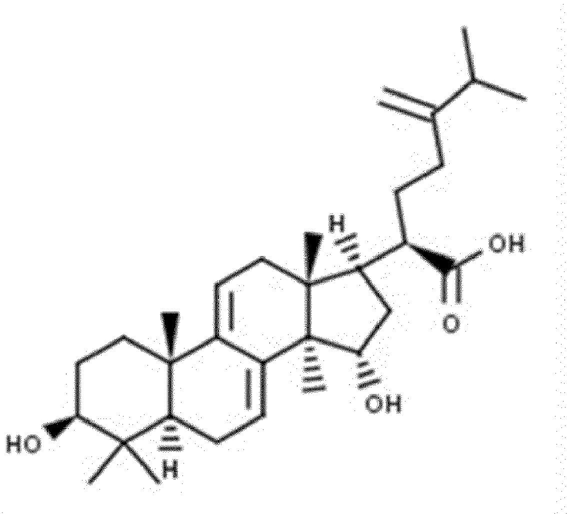

### Example 2: Efficacy Experiment

### Experimental animals

In the experiment, 7-week-old male SD rats purchased from LASCO Taiwan Co., Ltd. were used, which were domesticated and quarantined for 1 week. The rats were used for ischemic stroke assessment.

### Rearing environment

The rats were reared in the biomedical experimental animal station of the Industrial Technology Research Institute. The illumination time of the rearing area was automatically controlled for 12 hours bright, 12 hours dark, room temperature: 23 ± 2 °C, and relative humidity: 40-70%. The rats were free to get adequate food and water. During the quarantine and testing period, the veterinarians and test personnel of the institute performed observation and recordation daily to ensure the health status of the experimental animals.

### Animals observation

Clinical observations were made daily during the tests and recorded if the animals had other clinical symptoms or death. The clinical symptoms were observed as usual during holiday. Death and all abnormal symptoms (with different degrees of severity) were found and recorded in the animal clinical symptom observation record. Dead rats may also be under dissection to find possible causes of death.

### Animal grouping and individual identification

After 1 week of domestication on the experimental animals, the rats in good health condition were chosen. After weighing them, S-type grouping were performed. With 3 animals in one feeding cage, a number was tagged on the ears to distinguish between the experimental rats. Cage cards were pasted to mark cage numbers, strains, week ages, animal numbers, test numbers, test groups, admission dates, and test periods.

### 1. Animal model experiment for inducing ischemic stroke (MCAO)

### Middle Cerebral Artery Occlusion, MCAO / Reperfusion model

Test animals (250-350 g of male SD rats in this example) were anesthetized with 2% isoflurane in N2O / O2 (70% / 30%). The right common carotid artery (right CCA), external carotid artery (ECA), and internal carotid artery (ICA) were isolated.

Cutting along the midline of the scalp, the nylon monofilament (the front of the nylon monofilament was covered with polysiloxane) was inserted through the external carotid artery along the internal carotid artery and extending to circle of Willis of the brain, resulting in obstruction of the middle cerebral artery. After an hour of ischemia, the nylon monofilament was removed and the brain's blood was reperfused. After 24 hours, the brain of the rat was taken out and sliced, each having a thickness of 2 mm and with total 7 slices, so as to perform brain embolism area analysis.

### Experimental design and grouping

Two batches of experiments were performed with each batch being divided into four groups, each group having 5 rats. There were 40 rats in total.

In one embodiment of the present invention, a prevention mode experiment was adopted as shown in Fig. 1. At 10 minutes before the implementation of Animal model experiment for inducing ischemic stroke (MCAO), each of the test animals is administered with compounds: AR-04-41S, AR-04-15S, or AR-04-1822S respectively (50mg / kg) as described above.

The above-mentioned prevention model experiment further comprised a blank control group (sham) which were undergone no MCAO surgery and administered without the compound; and a vehicle control group (Vehicle) which was undergone MCAO surgery and administered with water in place of the compound.

### Results

### I. Analysis of neurobehavioral assessment

Neurobehavioral assessment in one embodiment of the present invention was performed on the rats at 0.5, 1.5 and 24 hours after MCAO surgery on each group of rats, and scored according to the following states. The purpose of the analytical assessment was to assess the severity of brain damage on rats.

Score 0: lifting the rat by its tail to about 20 to 30 cm above the ground and observing the state of stretching the forelimbs, at which state the forelimb of the rat could stretch towards the ground with balance and no occurrences of other nerve injuries is shown in Fig. 2 (A), representing the normal rat.
Score 1: lifting the rat by its tail to about 20 to 30 cm above the ground and observing the state of stretching the forelimb. The contraction of the forelimbs towards the contralateral of damaged area of the brain was shown in Fig. 2 (B).
Score 2: the rat was placed on the ground and a lateral thrust was applied. The resistance of the rat to the thrust ipsilateral to the damaged area of the brain was decreased. The experimental method was shown in Fig. 2 (C).
Score 3: the rat continued to go around in circles towards the contralateral side of damaged area of the brain and incapable of going straight when it was set free to exercise.
Score 4: due to severe nerve injury, limbs of the rat showed paralysis or epilepsy.

The results of neurobehavioral assessment at 0.5, 1.5, and 24 hours after MCAO surgery in each of the control and experimental groups were shown in Fig. 3. The Student's T test was adopted to determine whether there was difference between the groups administered with each compound and the Vehicle group. If the p value was less than or equal to 0.05, it represented significant difference. As shown in Fig. 3, the nerve injury became severe in at least 1.5 hours after MCAO surgery. At the time point of 24 hours, for the groups administered with the compounds of AR-04-15S, AR-04-1822S, and AR-04-41S, the nerve damages were all in recovery tendency, wherein the groups administered with AR-04-1822S and AR-04-41S were statistically significant in efficacy (p < 0.01).

### II. Analysis of brain infarct region

The brains of the rats were taken out at 24 hours after the MCAO surgery and placed in a low-temperature oxygenated physiological saline (0.95% normal saline). The coronal section of each of the brains was sliced into seven pieces with each piece having a thickness of 2mm. The forefront (1 mm) of the brain was removed. The brain tissue slices were then infiltrated with 1% 2,3,5-triphenyltetrazolium chloride (TTC) and reacted in an incubator of 37 °C for 30 minutes. The slices were fixed in 4% formalin solution and were recorded by a photographic system (MarcoPATH Digital Image System) as shown in Fig. 4A. The percentage of cerebral infarction volume as shown in Fig. 4B and fig. 4C was calculated by the image analysis software (ImageJ 1.42q).

Fig. 4B provides the brain infarct site and the percentage of the brain infarct volume of each compound and control group according to the embodiment in Fig. 4A, and the area of the infarct brain was analyzed from the front end to the range of 15 mm. Fig. 4C shows the total infarct volume (%) of the brain for each rat administered with each compound and control group according to the embodiment in Fig. 4A, and the area of the infarct brain was analyzed from the front end to the range of 15 mm. The Student's T test was adopted in the present embodiment. It was found in Fig. 4B and Fig. 4C that the group administered with the compound of No. AR-04-15S had a significant effect in reducing the infarct size at 7 mm, 9 mm and 11 mm from the front end of the brain; the group administered with the compound of No. AR-04-1822S had a significant effect in reducing the infarct size at 3 mm, 5 mm, 7mm, 9mm, 11mm, and 13 mm from the front end of the brain; and the group administered with the compound of No. AR-04-41S had a significant effect in reducing the infarct size at 3 mm, 7 mm, 9 mm, 11 mm, and 13 mm from the front end of the brain. Among them, the compound of No. AR-04-1822S provided a significantly better effect in reducing the infarct size at 7 mm and 9 mm from the front end of the brain, and the compound of No.AR-04-41S also provided a better efficacy at 7mm (p < 0.001).

As shown in Fig. 4C, the infarct volume (%) of the vehicle control group (Vehicle) was more than 30%; and all of the following compounds provided significant effects in reducing the infarct as compared with the vehicle control group (Vehicle) : No. AR-04-15S (p < 0.01), No. AR-04-1822S (p < 0.001), and No. AR-04-15S (p < 0.01). Among them, No. AR-04-41S (i.e. dehydroeburicoic acid as mentioned above) had the best effect on reducing the infarct volume (the infarct volume is less than 10%).

### III. Weight analysis

Table 1 below shows the changes in body weights of the rats during the operation of the middle cerebral artery occlusion, MCAO / reperfusion model before surgery (0 hr) and 24 hours (24 hr) after surgery.

**Table 1. Weight change table**

| **AVG** | | |
|---|---|---|
| **Group name** | **0hr** | **24hr** |
| Vehicle | 299.1 | 234.9 |
| 50 mg/kg AR-04-15S | 302.1 | 243.2 |
| 50 mg/kg AR-04-1822S | 311.3 | 270.0 |
| 50 mg/kg AR-04-41S | 314.2 | 266.5 |
| Sham | 353.7 | 328.7 |

| **SEM** | | |
|---|---|---|
| **Group name** | **0hr** | **24hr** |
| Vehicle | 13.2 | 7.0 |
| 50 mg/kg AR-04-15S | 14.1 | 5.6 |
| 50 mg/kg AR-04-1822S | 13.1 | 9.9 |
| 50 mg/kg AR-04-41S | 8.5 | 8.0 |
| Sham | 1.4 | 2.0 |

| **T-test** | | |
|---|---|---|
| **Group name** | **0hr** | **24hr** |
| Vehicle | - | - |
| 50 mg/kg AR-04-15S | 0.882 | 0.385 |
| 50 mg/kg AR-04-1822S | 0.532 | 0.023 |
| 50 mg/kg AR-04-41S | 0.370 | 0.018 |
| Sham | 0.014 | 0.000 |

In Table 1, AVG means average value, SEM means standard error of the mean, and T-test means Student's T test, which can be also referred to Fig. 5. Fig. 5 shows an analytical chart of body weight changes in each control and experimental group before MCAO surgery and 24 hours after surgery in one embodiment of the present invention. As shown in Fig. 5, the body weight of the rats decreased after cerebral ischemia, wherein the groups administered with the compounds of No. AR-04-1822S (p < 0.05) and No. AR-04-15S (p < 0.05) had significant retardation in body weight decrease as compared with the vehicle control group (Vehicle).

In summary, dehydroeburicoic acid (No. AR-04-41S), 4,7-Dimethoxy-5-methyl-1,3-benzodioxole (No. AR-04-15S), and dehydrosulphurenic acid (dehydrosulfurenic acid) provided a significant effects in reducing nerve injury in the rats after 24 hours from the MCAO surgery, and also in reducing the brain infarct volume caused by MCAO surgery. Among them, dehydroeburicoic acid (No. AR-04-41S) at the dose of 50 mg/kg and dehydrosulphurenic acid (dehydrosulfurenic acid) (No. AR-04-1822S) at the dose of 50 mg/kg had statistically significant effect in reducing nerve injury as compared with the vehicle control group (Vehicle). All of dehydroeburicoic acid (No. AR-04-41S) at the dose of 50 mg/kg, dehydrosulphurenic acid (dehydrosulfurenic acid) (No. AR-04-1822S) at the dose of 50 mg/kg, and 4,7-Dimethoxy-5-methyl-1,3-benzodioxole (No. AR-04-15S) at the dose of 50 mg/kg had statistically significant effect in reducing the brain infarct volume caused by MCAO surgery in the rats as compared with the vehicle control group (Vehicle).

While the present invention has been disclosed by way preferred embodiments, it is not intended to limit the present invention. Any person of ordinary skill in the art may, without departing from the spirit and scope of the present invention, shall be allowed to perform modification and embellishment. Therefore, the scope of protection of the present invention shall be governed by which defined by the claims attached subsequently.

## Claims

1. A compound for use in treating stroke or reducing nerve injury, which is selected from the group consisting of the following:
(1) 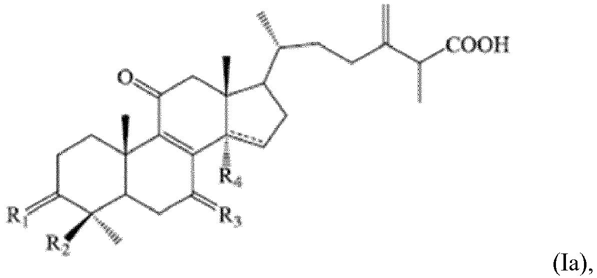 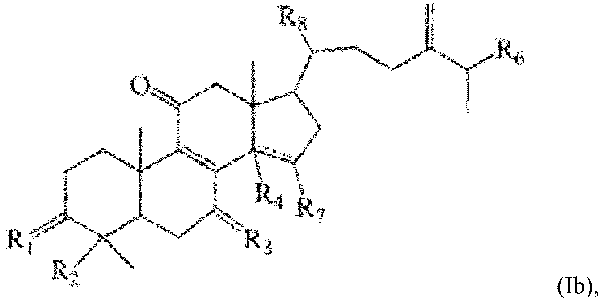 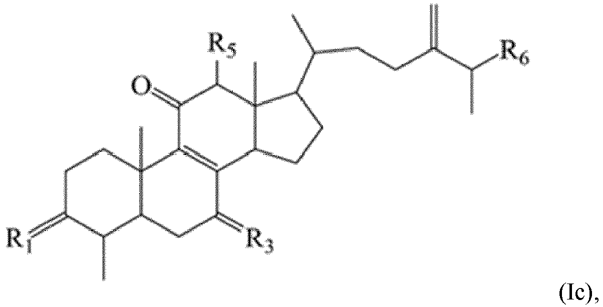 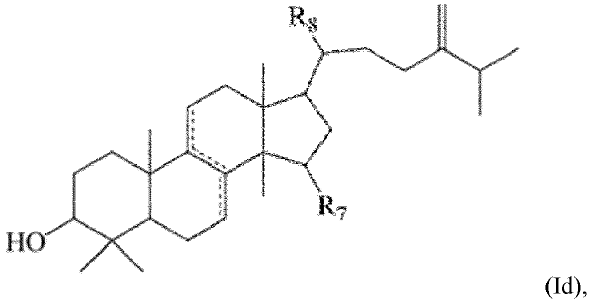 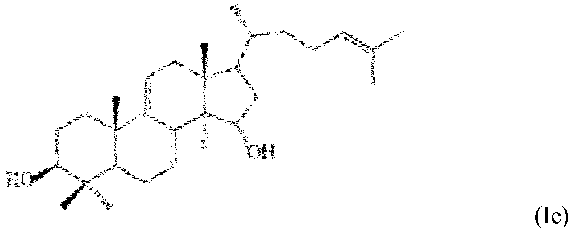 wherein R₁ is O, α-OH or β-H; R₂ is H or OH; R₃ is O, α-H, β-OAc or H₂; R₄ is H or OH; R₅ is H, or OH; R₆ is COOH or COO(CH₂)n-CH₃; R₇ is H, OH or OAc; R₈ is CH₃ or COOH; the dotted line represents a single bond or a double bond; n is an integer from 0-3; or
(2) 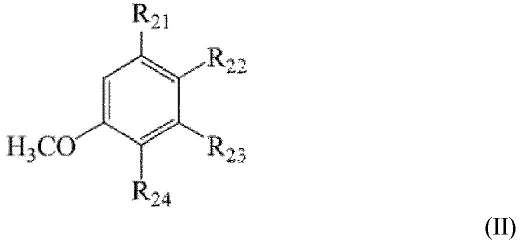 wherein R₂₁ is CH₃, COOH, or COO(CH₂)n-CH₃; n is an integer from 0-3; each of R₂₂, R₂₃, R₂₄ is OCH₃, or R₂₂ and R₂₃ together form a O-CH₂-O; or R₂₃ and R₂₄ together form a O-CH₂-O.

2. The compound for use in treating stroke or reducing nerve injury of claim 1, wherein the compound is selected from the group consisting of: 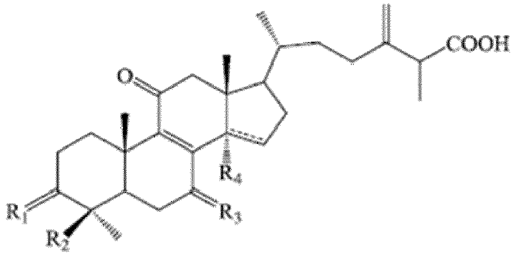 wherein R₁ is O or α-OH; R₂ is H or OH; R₃ is O, β-OH or H₂; R₄ is H or OH; the dotted line represents a single bond or a double bond.

3. The compound for use in treating stroke or reducing nerve injury of claim 1, wherein the compound is selected from the group consisting of: 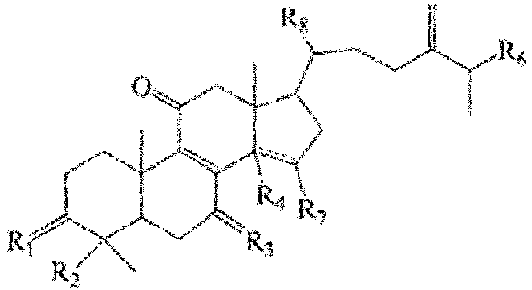 wherein R₁ is O or α-OH; R₃ is O, β-OH or H₂; R₄ is H; the dotted line represents a single bond or a double bond; R₅ is H or OH; R₆ is COOH or COOEt; R₇ is H, OH or OAc; R₈ is CH₃ or COOH; the dotted line represents a single bond or a double bond.

4. The compound for use in treating stroke or reducing nerve injury of claim 1, wherein the compound is selected from the group consisting of: 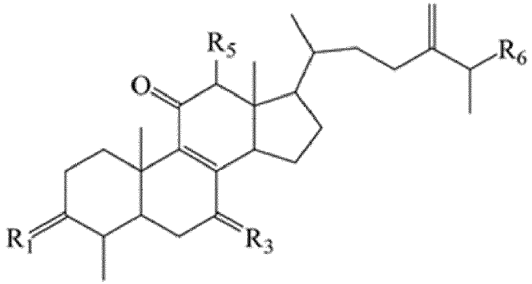 wherein R₁ is O, α-OH or β-H; R₃ is O, α-H, β-OAc or H₂; R₅ is H, or OH; R₆ is COOMe.

5. The compound for use in treating stroke or reducing nerve injury of claim 1, wherein the compound is selected from the group consisting of: 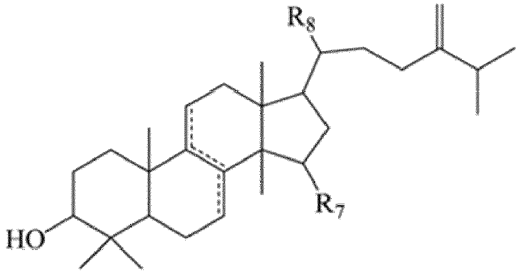 wherein R₇ is H, OH or OAc; R₈ is CH₃ or COOH; the dotted line represents a single bond or a double bond.

6. The compound for use in treating stroke or reducing nerve injury of claim 1, wherein the compound is selected from the group consisting of:

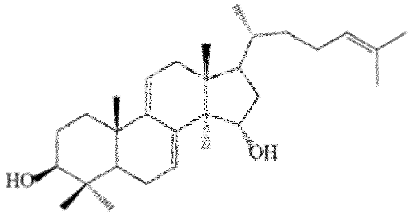

7. The compound for use in treating stroke or reducing nerve injury of claim 1, wherein the compound of formula (Ia) is selected from the group consisting of: 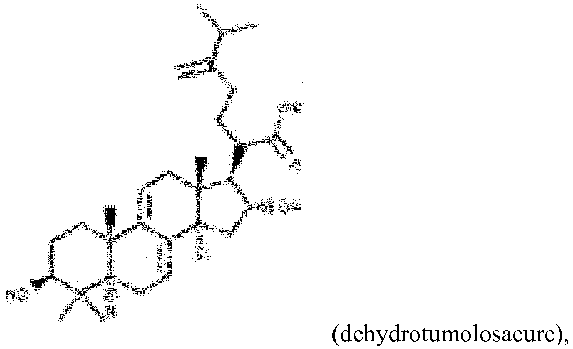 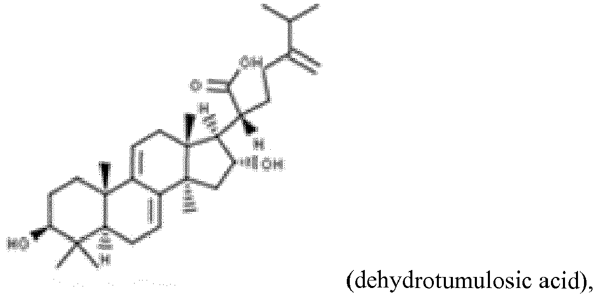 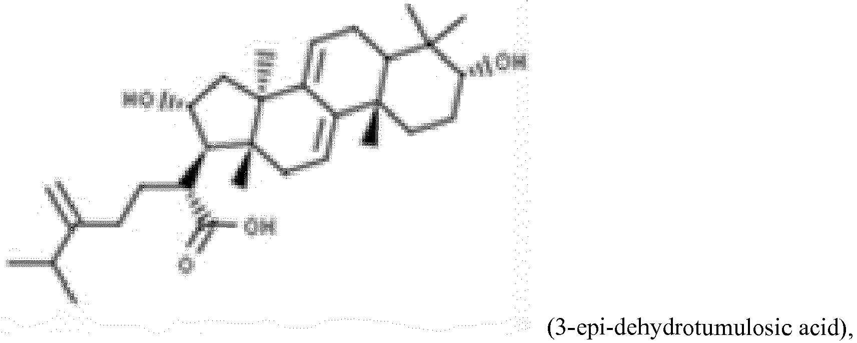 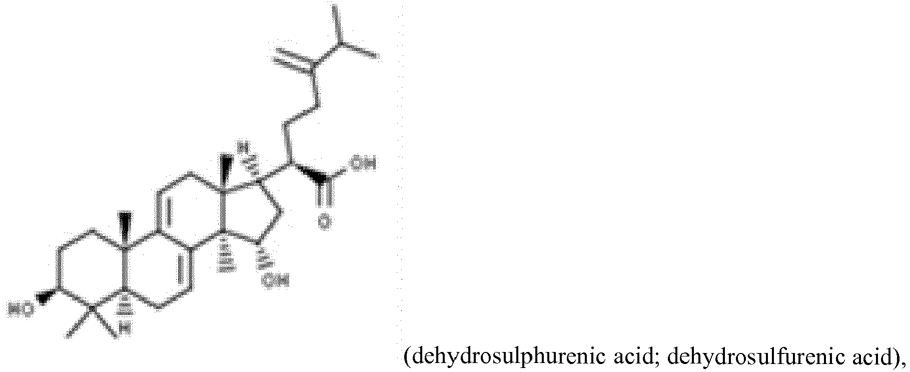 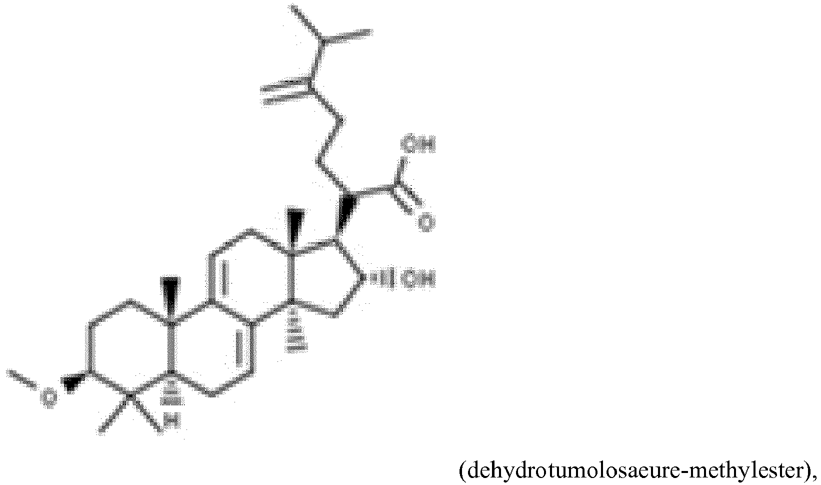 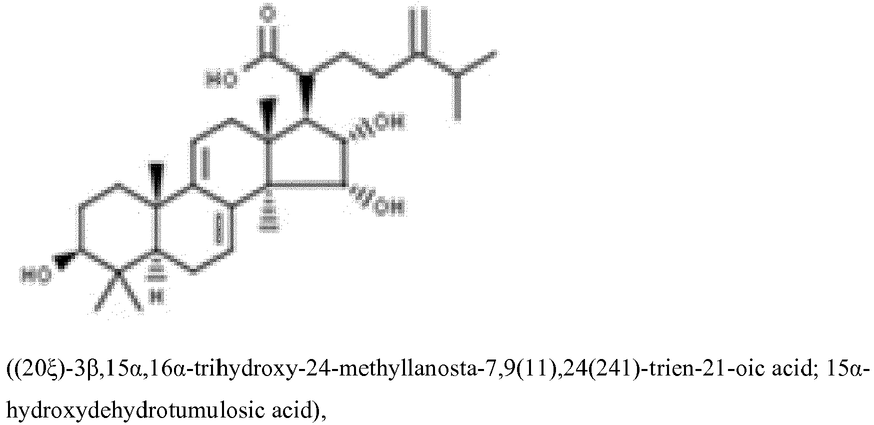 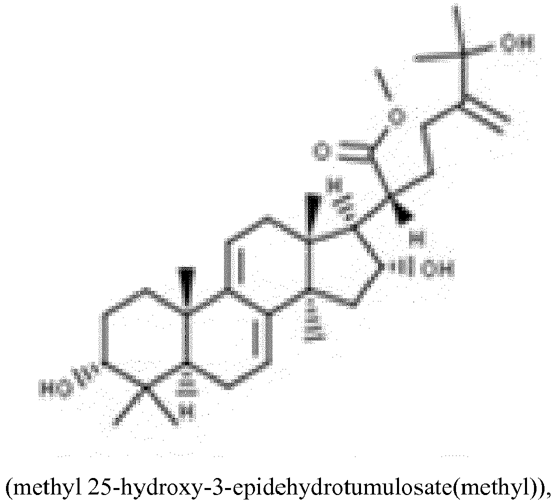 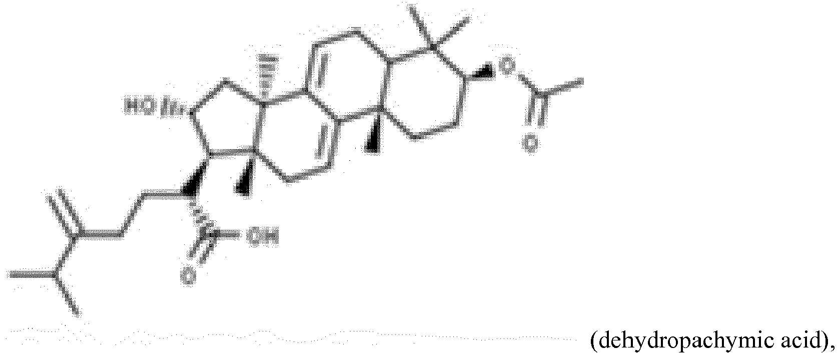 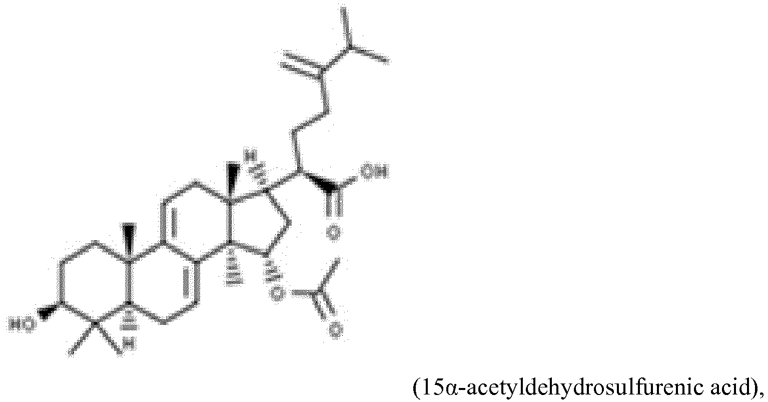 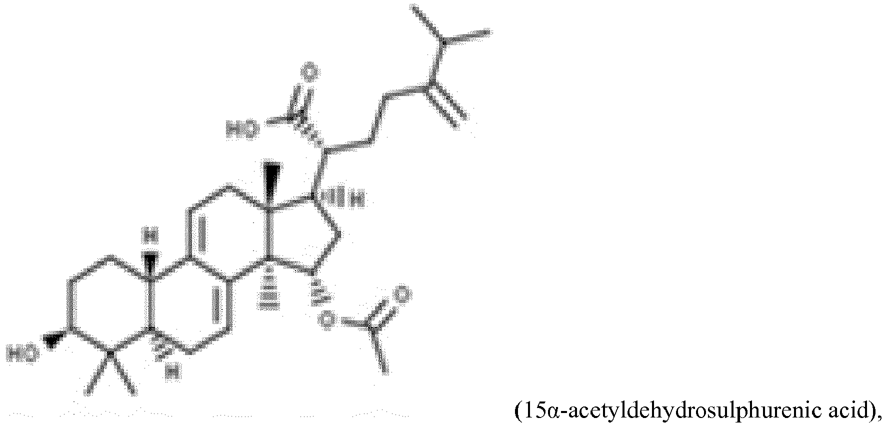 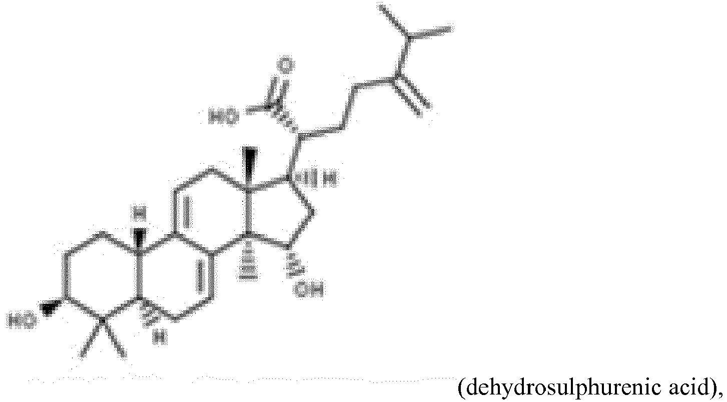 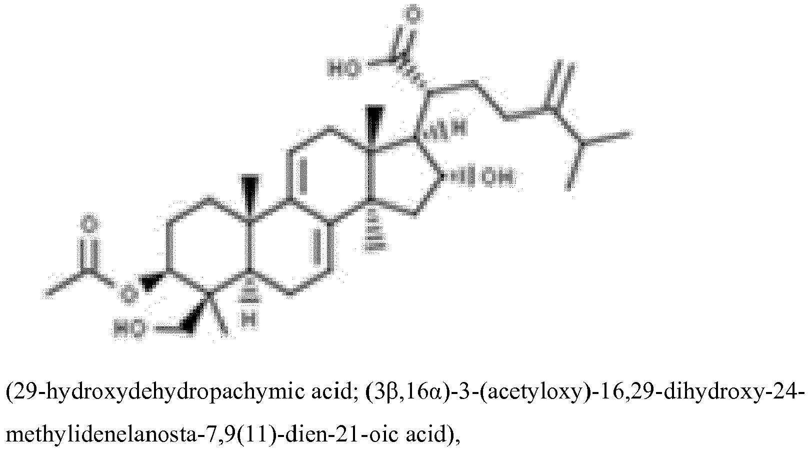 and

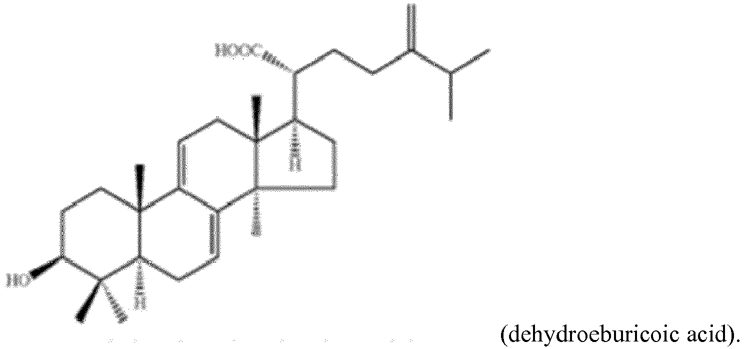

8. The compound for use in treating stroke or reducing nerve injury of claim 1, wherein the compound of formula (Ib) is selected from the group consisting of 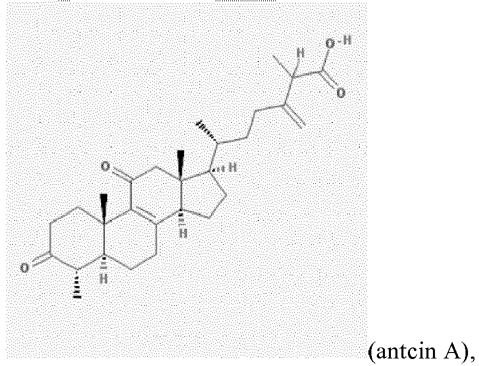 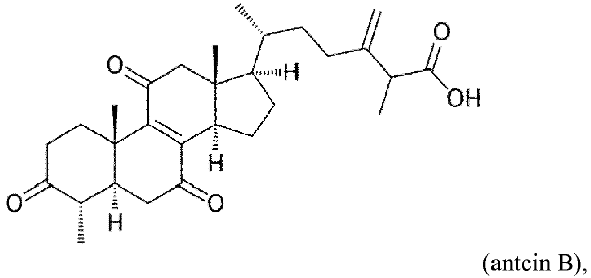 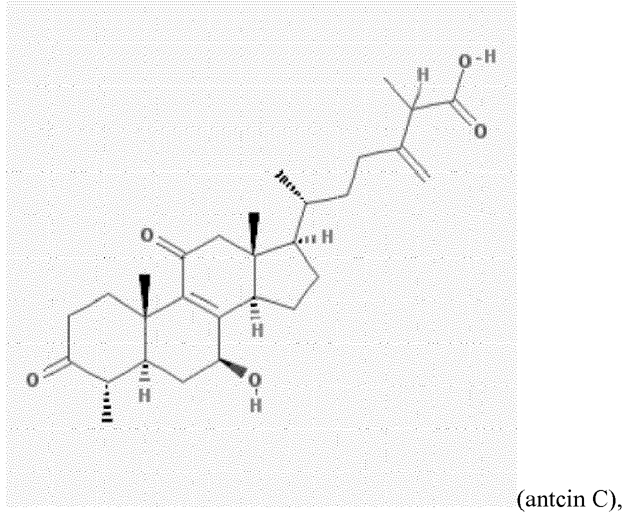 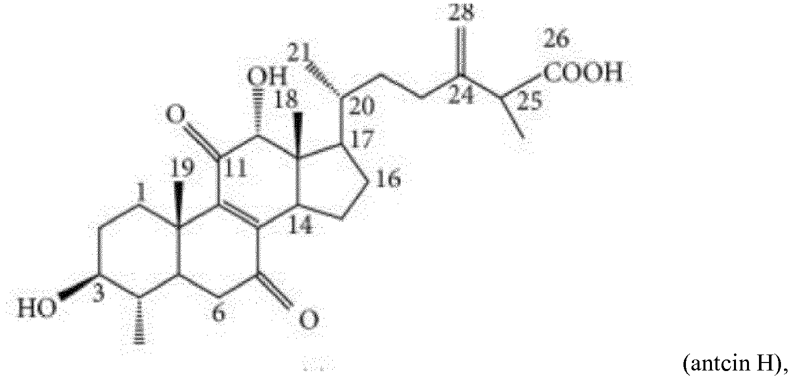 or

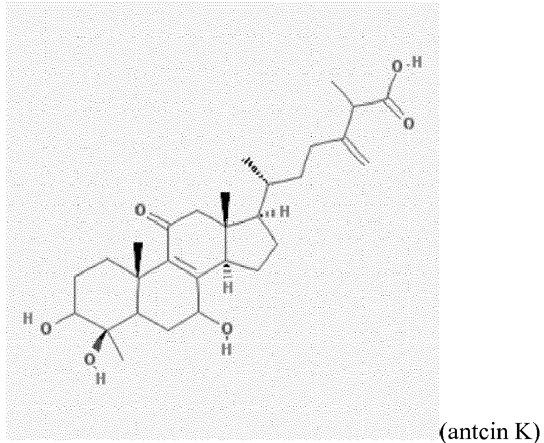

9. The compound for use in treating stroke or reducing nerve injury of claim 1, wherein the compound of formula (II) is:

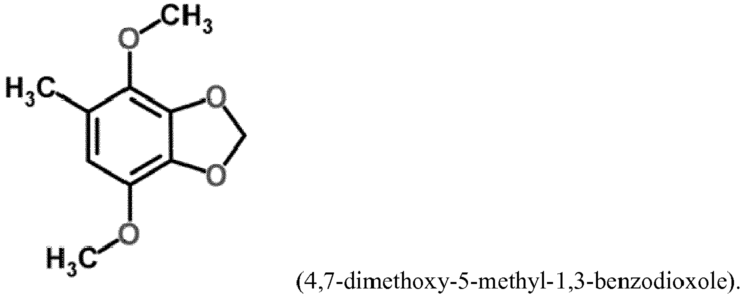

10. The compound for use in treating stroke or reducing nerve injury of claim 1, wherein the compound is dehydroeburicoic acid, 4,7-Dimethoxy-5-methyl-1,3-benzodioxole or dehydrosulphurenic acid (dehydrosulfurenic acid).

11. The compound for use in treating stroke or reducing nerve injury of claim 1, wherein the stroke is ischemia stroke.

12. The compound for use in treating stroke or reducing nerve injury of claim 1, wherein the compound is dehydroeburicoic acid.

13. The compound for use in treating stroke or reducing nerve injury of claim 1, wherein the compound is 4,7-Dimethoxy-5-methyl-1,3-benzodioxole.

14. The compound for use in treating stroke or reducing nerve injury of claim 1, wherein the compound is dehydrosulphurenic acid (dehydrosulfurenic acid).
